(19) Europäisches Patentamt
European Patent Office
Office européen des brevets

(11) EP 2 397 129 A1

(12) EUROPEAN PATENT APPLICATION

(43) Date of publication:
21.12.2011 Bulletin 2011/51

(21) Application number: 11180387.0

(22) Date of filing: 06.07.2005

(51) Int Cl.:
*A61K 31/216* (2006.01) *A61P 3/04* (2006.01)
*A61P 3/10* (2006.01) *A61P 9/10* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **16.07.2004 JP 2004210492**
**02.08.2004 US 598233 P**

(62) Document number(s) of the earlier application(s) in accordance with Art. 76 EPC:
**05765510.2 / 1 769 793**

(71) Applicant: **Japan Tobacco, Inc.**
**Tokyo 105-8422 (JP)**

(72) Inventors:
- **Furukawa, Noboru**
  **Takatsuki-shi, Osaka 5691125 (JP)**
- **Mera, Kawai**
  **Takatsuki-shi, Osaka 5691125 (JP)**
- **Matsushita, Mutsuyoshi**
  **Takatsuki-shi, Osaka 5691125 (JP)**
- **Ogawa, Naoto**
  **Takatsuki-shi, Osaka 5691125 (JP)**
- **Sumida, Yukako**
  **Takatsuki-shi, Osaka 5691125 (JP)**
- **Okuma, Chihiro**
  **Takatsuki-shi, Osaka 5691125 (JP)**
- **Hata, Takahiro**
  **Takatsuki-shi, Osaka 5691125 (JP)**

(74) Representative: **Vossius & Partner**
**Siebertstrasse 4**
**81675 München (DE)**

Remarks:
This application was filed on 07-09-2011 as a divisional application to the application mentioned under INID code 62.

# (54) Agent for the treatment or prevention of diabetes, obesity or arteriosclerosis

(57) Because the compound of the invention represented by the formula (1):

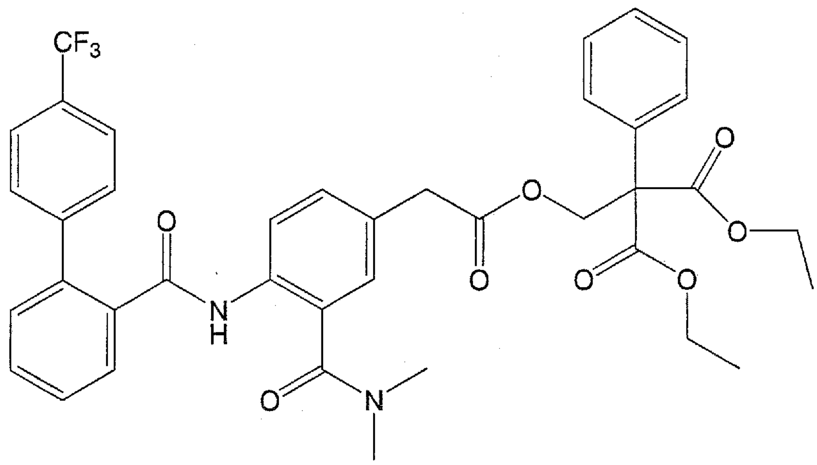

(1)

or its pharmaceutically acceptable salt has excellent antidiabetic effect, anti-obesity effect and anti-arteriosclerosis effect, a medicine containing the compound of formula (1) as the active ingredient is useful as an agent for the prevention or treatment of diabetes (especially non-insulin dependent diabetes mellitus), obesity (especially visceral fat obesity), or arteriosclerosis.

EP 2 397 129 A1

## Description

Technical Field

The present invention relates to an agent for the treatment or prevention of diabetes, obesity or arteriosclerosis.

Background Art

Diabetes is a representative adult disease in which blood sugar level is difficult to be controlled, and is often complicated with peripheral neuropathy or the like. Diabetes is classified into insulin-dependent diabetes mellitus (hereinafter referred to as type 1 diabetes) and non-insulin dependent diabetes mellitus (hereinafter referred to as type 2 diabetes). Most Japanese diabetics are of the type 2 diabetes. Type 2 diabetes is more severely affected by lifestyle and aging than type 1 diabetes, and is apt to occur in obese people of middle-age or older.

Obesity is a condition characterized by the systemic increase of adipose tissues as a result of the long-term energy intake exceeding the energy expenditure. Obesity is classified into two categories: visceral fat obesity and subcutaneous fat obesity. Visceral fat obesity is a type of obesity with increased accumulation of intra-abdominal fat around the greater omentum and the mesenterium, and is said to be the main culprit behind diabetes (particularly type 2 diabetes accompanied by insulin resistance), arteriosclerosis, liver disease, heart disease, etc., which is a big problem in the modern society.

Arteriosclerosis is generally a condition in which fat and calcium salt are built up on the locally thickened and proliferated arterial walls , resulting in destruction of elastic fibers of the blood vessel walls and loss of elasticity of blood vessel, and this is categorized as a lifestyle-related disease. Risk factors of arteriosclerosis are not only serum cholesterol and thrombi but also many others including obesity and diabetes.

As mentioned above, arteriosclerosis, obesity, and diabetes are closely related with each other, and various pharmaceutical agents, in addition to dietetic therapy and therapeutic exercise, have been used for the treatment and prevention of those diseases.

International Publication WO 2003/0725322 discloses a compound represented by the formula:

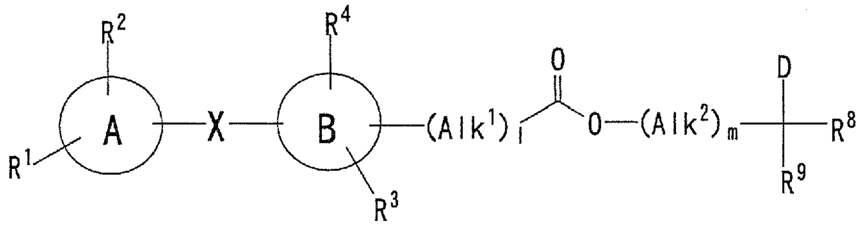

and describes that the compound is useful as a therapeutic agent for hyperlipidemia based on its MTP-inhibiting effect, apo-B secretion-inhibiting effect, triglyceride-lowering effect, and the like.

Disclosure of the Invention

The present invention aims at providing an agent having an excellent antidiabetic effect, anti-obesity effect and anti-arteriosclerosis effect for the treatment or prevention of diabetes (type 2 diabetes in particular), obesity (visceral fat obesity in particular), or arteriosclerosis.

The inventors, as the result of intensive researches to solve the above-mentioned problems, have found that the compound represented by the formula (1):

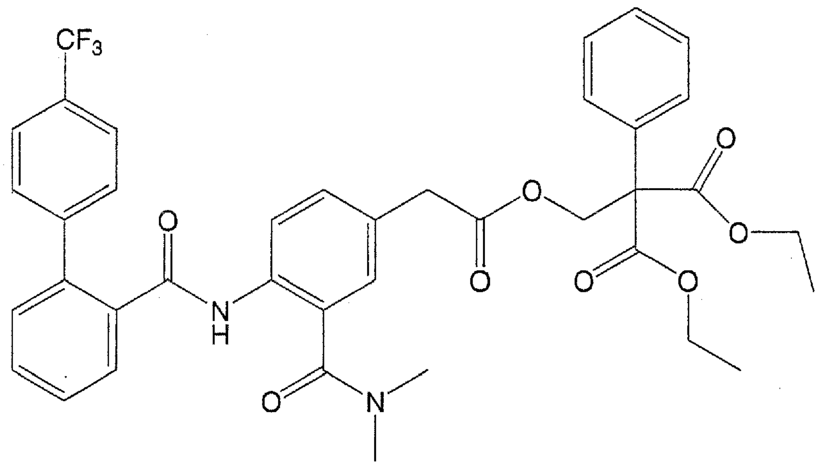

(1)

among the wide-ranging compounds having the ester structure described in said International Publication WO 2003/0725322 has, unexpectedly, a very excellent antidiabetic effect (anti-type 2 diabetes effect in particular), anti-obesity effect (anti-visceral fat obesity effect in particular) and anti-arteriosclerosis effect, and thus completed the present invention.

Namely, the invention relates to

(1) an agent for the prevention or treatment of diabetes, comprising a compound represented by the formula (1):

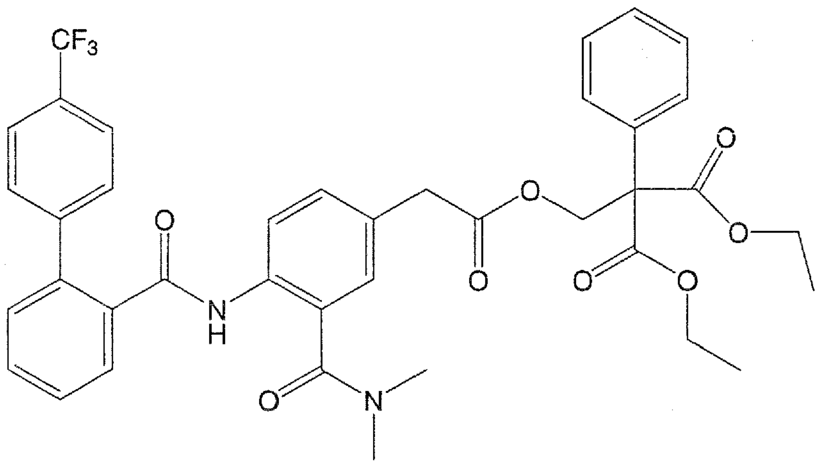

(1)

or its pharmaceutically acceptable salt as an active ingredient;
(2) the agent for the prevention or treatment described in the above (1), wherein the diabetes is non-insulin dependent diabetes mellitus;
(3) the agent for the prevention or treatment described in the above (2), wherein the diabetes is accompanied by obesity or/and arteriosclerosis;
(4) the agent for the prevention or treatment described in the above (3), which suppresses obesity or/and arteriosclerosis;
(5) the agent for the prevention or treatment described in the above (3) or (4), wherein the obesity is visceral fat obesity;
(6) the agent for the prevention or treatment described in any one of the above (2) to (5), wherein the non-insulin dependent diabetes mellitus is insulin-resistant;
(7) the agent for the prevention or treatment described in any one of the above (1) to (6), which causes no induction of liver dysfunction;
(8) the agent for the prevention or treatment described in any one of the above (1) to (7), wherein the unit dose of the compound represented by the formula (1) or its pharmaceutically acceptable salt is 0.5 to 30 mg and the daily dose is 0.5 to 30 mg;

(9) an agent for the prevention or treatment of obesity, comprising a compound represented by the formula (1):

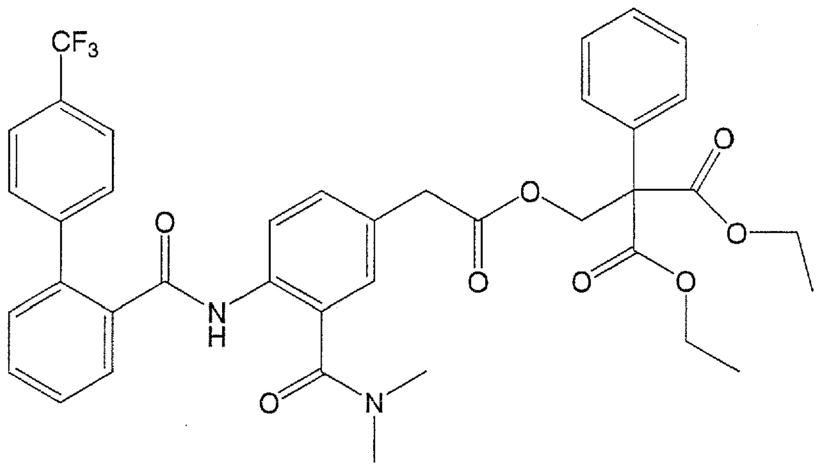

(1)

or its pharmaceutically acceptable salt as an active ingredient;
(10) the agent for the prevention or treatment described in the above (9), wherein the obesity is visceral fat obesity;
(11) the agent for the prevention or treatment described in the above (10), wherein the visceral fat obesity is accompanied by non-insulin dependent diabetes mellitus or/and arteriosclerosis;
(12) the agent for the prevention or treatment described in the above (11), which suppresses non-insulin dependent diabetes mellitus or/and arteriosclerosis;
(13) the agent for the prevention or treatment described in the above (11) or (12), wherein the non-insulin dependent diabetes mellitus is insulin-resistant;
(14) the agent for the prevention or treatment described in any one of the above (9) to (13), which causes no induction of liver dysfunction;
(15) the agent for the prevention or treatment described in any one of the above (9) to (14), wherein the unit dose of the compound represented by the formula (1) or its pharmaceutically acceptable salt is 0.5 to 30 mg and the daily dose is 0.5 to 30 mg;
(16) an agent for the prevention or treatment of arteriosclerosis, comprising a compound represented by the formula (1):

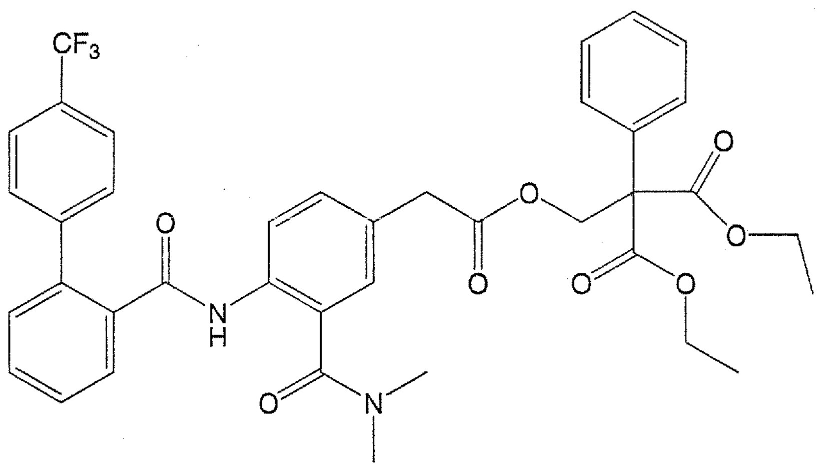

(1)

or its pharmaceutically acceptable salt as an active ingredient;
(17) the agent for the prevention or treatment described in the above (16), wherein the arteriosclerosis is accompanied by non-insulin dependent diabetes mellitus or/and visceral fat obesity;
(18) the agent for the prevention or treatment described in the above (17), which suppresses non-insulin dependent diabetes mellitus or/and visceral fat obesity;

(19) the agent for the prevention or treatment described in the above (17) or (18), wherein the non-insulin dependent diabetes mellitus is insulin-resistant;
(20) the agent for the prevention or treatment described in any one of the above (16) to (19), which causes no induction of liver dysfunction; and
(21) the agent for the prevention or treatment described in any one of the above (16) to (20), wherein the unit dose of the compound represented by the formula (1) or its pharmaceutically acceptable salt is 0.5 to 30 mg and the daily dose is 0.5 to 30 mg.

The present invention also relates to (22) use of a compound represented by the formula (1):

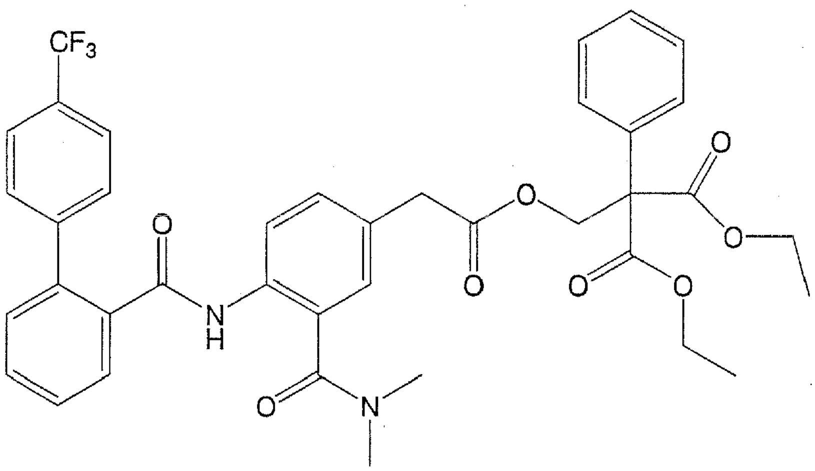

(1)

or its pharmaceutically acceptable salt for preparing a medicine for the prevention or treatment of diabetes;

(23) the use described in the above (22), wherein the diabetes is non-insulin dependent diabetes mellitus;
(24) the use described in the above (23), wherein the non-insulin dependent diabetes mellitus is accompanied by obesity or/and arteriosclerosis;
(25) the use described in the above (24), which suppresses obesity or/and arteriosclerosis;
(26) the use described in the above (24) or (25), wherein the obesity is visceral fat obesity;
(27) the use described in any one of the above (23) to (26), wherein the non-insulin dependent diabetes mellitus is insulin-resistant;
(28) the use described in any one of the above (22) to (27), which causes no induction of liver dysfunction;
(29) the use described in any one of the above (22) to (28), wherein the unit dose of the compound represented by the formula (1) or its pharmaceutically acceptable salt is 0.5 to 30 mg and the daily dose is 0.5 to 30 mg;
(30) use of a compound represented by the formula (1):

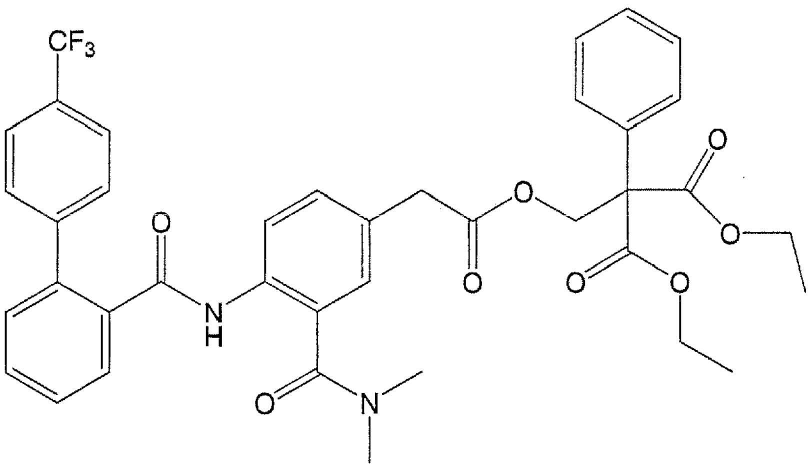

(1)

or its pharmaceutically acceptable salt for preparing a medicine for the prevention or treatment of obesity;
(31) the use described in the above (30), wherein the obesity is visceral fat obesity;
(32) the use described in the above (31), wherein the visceral fat obesity is accompanied by non-insulin dependent diabetes mellitus or/and arteriosclerosis;
(33) the use described in the above (32), which suppresses non-insulin dependent diabetes mellitus or/and arteriosclerosis;
(34) the use described in the above (32) or (33), wherein the non-insulin dependent diabetes mellitus is insulin-resistant;
(35) the use described in any one of the above (30) to (34), which causes no induction of liver dysfunction;
(36) use described in any one of the above (30) to (35), wherein the unit dose of the compound represented by the formula (1) or its pharmaceutically acceptable salt is 0.5 to 30 mg and the daily dose is 0.5 to 30 mg;
(37) the use of the compound represented by the formula (1):

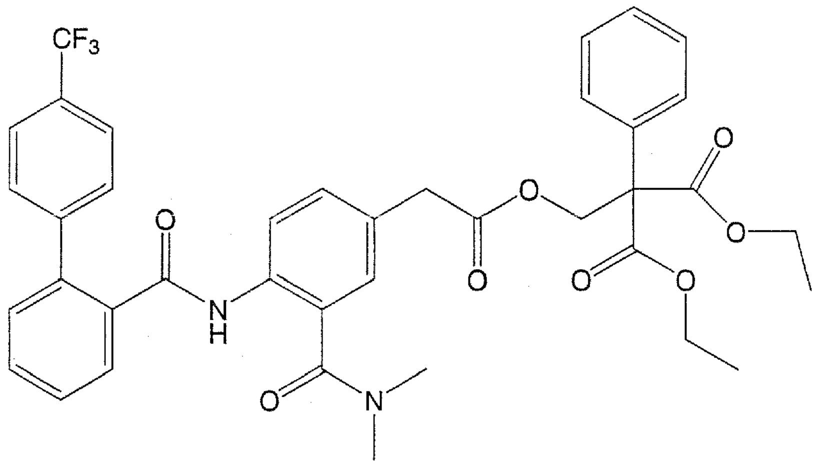

(1)

or its pharmaceutically acceptable salt for preparing a medicine for the prevention or treatment of arteriosclerosis;
(38) the use described in the above (37), wherein the arteriosclerosis is accompanied by non-insulin dependent diabetes mellitus or/and visceral fat obesity;
(39) the use described in the above (38), which suppresses non-insulin dependent diabetes mellitus or/and arteriosclerosis;
(40) the use described in the above (38) or (39), wherein the non-insulin dependent diabetes mellitus is insulin-resistant;
(41) the use described in any one of the above (37) to (40), which causes no induction of liver dysfunction;
(42) the use described in any one of the above (37) to (41), wherein the unit dose of the compound represented by the formula (1) or its pharmaceutically acceptable salt is 0.5 to 30 mg and the daily dose is 0.5 to 30 mg.

The invention also relates to:

(43) a method for the prevention or treatment of diabetes, which comprises administering a compound represented by the formula (1):

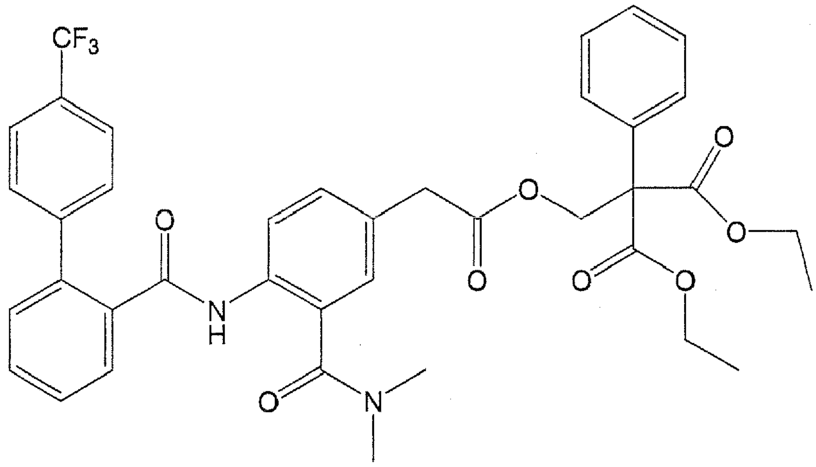

(1)

or its pharmaceutically acceptable salt to a mammal;

(44) the method for the prevention or treatment described in the above (43), wherein the diabetes is non-insulin dependent diabetes mellitus;

(45) the method for the prevention or treatment described in the above (44), wherein the non-insulin dependent diabetes mellitus is accompanied by obesity or/and arteriosclerosis;

(46) the method for the prevention or treatment described in the above (45), which suppresses obesity or/and arteriosclerosis;

(47) the method for the prevention or treatment described in the above (45) or (46), wherein the obesity is visceral fat obesity;

(48) the method for the prevention or treatment described in any one of the above (44) to (47), wherein the non-insulin dependent diabetes mellitus is insulin-resistant;

(49) the method for the prevention or treatment described in any one of the above (43) to (48), which causes no induction of liver dysfunction;

(50) the method for the prevention or treatment described in any one of the above (43) to (49), wherein the unit dose of the compound represented by the formula (1) or its pharmaceutically acceptable salt is 0.5 to 30 mg and the daily dose is 0.5 to 30 mg;

(51) a method for the prevention or treatment of obesity, which comprises administering a compound represented by the formula (1):

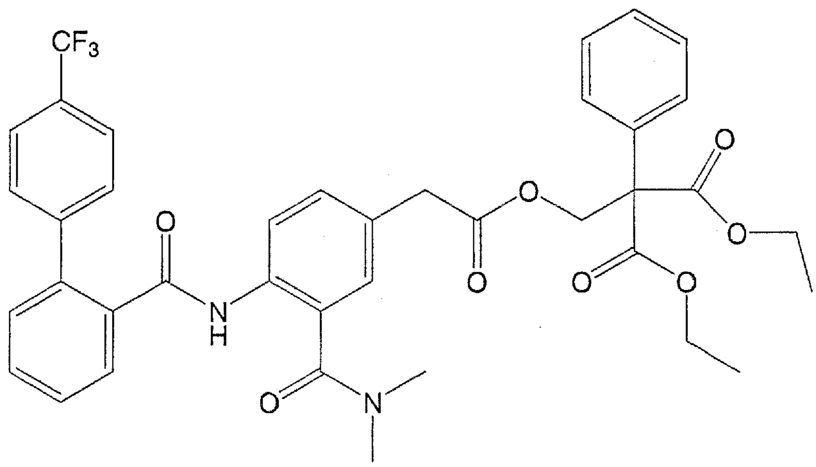

(1)

or its pharmaceutically acceptable salt to a mammal;

(52) the method for the prevention or treatment described in the above (51), wherein the obesity is visceral fat obesity;

(53) the method for the prevention or treatment described in the above (52), wherein the visceral fat obesity is accompanied by non-insulin dependent diabetes mellitus or/and arteriosclerosis;

(54) the method for the prevention or treatment described in the above (53), which suppresses non-insulin dependent

diabetes mellitus or/and arteriosclerosis;
(55) the method for the prevention or treatment described in the above (53) or (54), wherein the non-insulin dependent diabetes mellitus is insulin-resistant;
(56) the method for the prevention or treatment described in any one of the above (51) to (55), which causes no induction of liver dysfunction;
(57) the method for the prevention or treatment described in any one of the above (51) to (56), wherein the unit dose of the compound represented by the formula (1) or its pharmaceutically acceptable salt is 0.5 to 30 mg and the daily dose is 0.5 to 30 mg;
(58) a method for the prevention or treatment of arteriosclerosis, which comprises administering a compound represented by the formula (1):

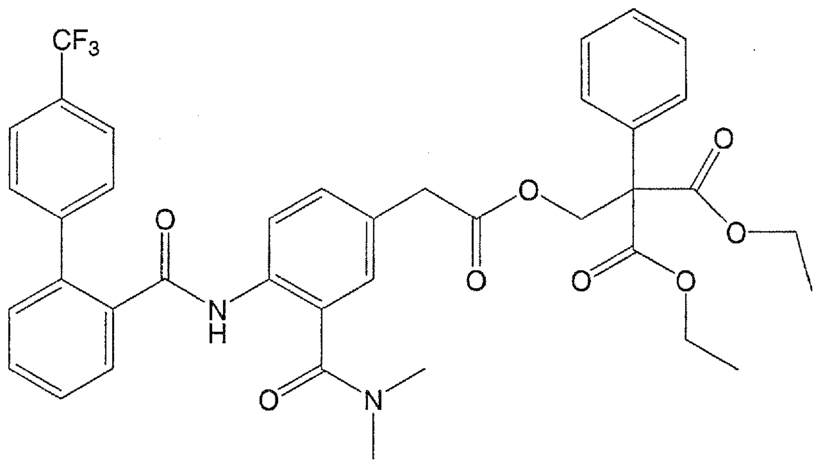
(1)

or its pharmaceutically acceptable salt to a mammal;
(59) the method for the prevention or treatment described in the above (58), wherein the arteriosclerosis is accompanied by non-insulin dependent diabetes mellitus or/and visceral fat obesity;
(60) the method for the prevention or treatment described in the above (59), which suppresses non-insulin dependent diabetes mellitus or/and visceral fat obesity;
(61) the method for the prevention or treatment described in the above (59) or (60), wherein the non-insulin dependent diabetes mellitus is insulin-resistant;
(62) the method for the prevention or treatment described in any one of the above (58) to (61), which causes no induction of liver dysfunction;
(63) the method for the prevention or treatment described in any one of the above (58) to (62), wherein the unit dose of the compound represented by the formula (1) or its pharmaceutically acceptable salt is 0.5 to 30 mg and the daily dose is 0.5 to 30 mg.

Effect of the Invention

The compound represented by the formula (1), the active ingredient of the present invention, has a very excellent antidiabetic effect, that is above all effective for type 2 diabetes, especially for insulin-resistant type 2 diabetes. In addition, the compound represented by the formula (1), the active ingredient of the present invention, has a very excellent anti-obesity effect, that is above all effective for visceral fat obesity. Furthermore, the compound represented by the formula (1), the active ingredient of the present invention, has a very excellent anti-arteriosclerosis effect. In particular, the compound represented by the formula (1), the active ingredient of the present invention, has effects for simultaneously preventing or treating diabetes (particularly type-2 diabetes, above all insulin-resistant type 2 diabetes), obesity (particularly visceral fat obesity), and/or arteriosclerosis at the same time. The compound represented by formula (1), the active ingredient of the present invention, can be used safely because it does not induce any specific adverse drug reaction such as liver dysfunction.

Best Mode for Carrying Out the Invention

The chemical name of the compound represented by formula (1), the active ingredient of the present invention, is 2-(2-{3-dimethylcarbamoyl-4-[(4'-trifluoromethyl-biphenyl-2-carbonyl)amino]phenyl}acetoxymethyl)-2-phenylmalanic

acid diethyl ester (abbreviated to Compound (1) hereinafter).

"Pharmaceutically acceptable salts" include various inorganic acid salts such as hydrochloride, hydrobromide, sulfate, phosphate, and nitrate; various organic acid salts such as acetate, propionate, succinate, glycolate, lactate, malate, oxalate, tartrate, citrate, maleate, fumarate, methanesulfonate, benzenesulfonate, p-toluenesulfonate, and ascorbate; and salts with various amino acids such as aspartate and glutamate, but are not limited thereto. The pharmaceutically acceptable salts of Compound (1), the active ingredient of the present invention, include intramolecular salts and adducts of Compound (1), and complexes, moisture-containing substances, solvates and hydrates thereof. Depending on the situation, metabolites thereof may be included.

Compound (1), the active ingredient of the present invention, is a known compound described in Examples 2 to 5 in the International Publication WO 2003/0725322 and can be manufactured according to the method described in the literature.

Compound (1), the active ingredient of the present invention, is administered systemically or locally, and orally or parenterally. The dose varies depending on the route of administration, age and body weight of the patient, symptom, therapeutic effect, etc., while it is generally in the range of 0.1 mg to 1 g, preferably in the range of 0.5 to 30 mg, once to several times a day, preferably once a day, for an adult. Compound (1), the active ingredient of the present invention, can be used for treatment or prevention of the above-mentioned diseases not only in human but also in animals, particularly in mammals.

Compound (1), the active ingredient of the present invention, can be manufactured into medicines for the prevention or treatment of the above-mentioned diseases together with, as required, pharmaceutically acceptable carriers and other additive. The dosage forms of the medicines for the prevention or treatment include tablets, pills, powders, granules, suppositories, injections, eye drops, liquid preparations, capsules, troches, aerosols, elixirs, suspensions, emulsions and syrups.

When the agent for the prevention or treatment of the present invention is prepared in the form of a solid preparation such as tablets, pills, powders, granules, etc., the usable additives include lactose, mannitol, glucose, hydroxypropylcellulose, microcrystalline cellulose, starch, polyvinylpyrrolidone, magnesium metasilicate aluminate, and silicic anhydride powder. When the agent of the invention is prepared in the form of tablets or pills, the agent may be coated with a film made from a gastro-soluble or enteric substance sucrose, gelatin, hydroxypropylcellulose or hydroxymethylcellulose phthalate, or may be prepared in multilayer tablets.

The manufacturing method of the agent for the prevention or treatment of the present invention in the form of a solid preparation such as granule or tablet is described below.

1. Granulation process

(1) A suitable amount of Compound (1) as the active ingredient is dissolved in a suitable organic solvent such as acetone, anhydrous ethanol, etc. or in a mixture thereof. The amount of the organic solvent should be sufficient enough to dissolve the water-soluble polymer to be added in the subsequent process. On the other hand, when an unnecessarily much solvent is used, not only the subsequent operations will become complicated but also some undesirable influence will be exerted on the preparation. The appropriate amount of the solvent used is one to two times the weight of the polymer. Dissolution by stirring can be performed using, for example, a propeller mixer.

(2) To the solution of a suitable amount of Compound (1) in an organic solvent is added a water-soluble polymer, followed by further mixing by stirring for complete dissolution of both the Compound (1) and the water-soluble polymer. The solvent may be heated to promote dissolution if necessary.

(3) Separately, an inorganic porous substance such as light anhydrous silicic acid as an excipient and, if necessary, a disintegrant such as calcium carboxymethylcellulose are mixed by stirring using a mixing stirrer or the like.

(4) The mixture of an inorganic porous substance and a disintegrant prepared in the above (3) and the solution of Compound (1) and a polymer are mixed, and then the resultant mixture is granulated according to the conventional method.

From the viewpoint of operationability, it is desirable to add the solution obtained in the above (2) to the mixture obtained in the above (3) before mixing, although the method of mixing is not limited thereto and mixing by addition of the mixture obtained in the above (3) to the solution obtained in the above (2) is also permissible.

(5) Next, the mixture is granulated, followed by evaporation of the organic solvent under reduced or atmospheric pressure by, for example, vacuum drying.

(6) Finally the dried granulates are sized using a sieve or the like to give granules.

The organic solvents used in the operation (1) are not limited as far as they can dissolve Compound (1) as the active ingredient and the polymer, including alcohols such as methanol, ethanol, propanol, isopropyl alcohol, butanol,

monomethoxyethanol and ethylene glycol monomethyl ether; ketones such as acetone and methylketone; and mixtures thereof. Solvents having a low boiling point, such as ketones exemplified by acetone and alcohols exemplified by anhydrous ethanol are desirable, and acetone, anhydrous ethanol, and mixtures thereof are particularly desirable. Water may be added to these solvents as needed.

2. Tableting process

When tablets are desired, a disintegrant such as calcium carboxymethylcellulose and cross-linked polyvinylpyrrolidone, and a lubricant such as magnesium stearate are added to the sized granulates obtained in the above-mentioned granulation process, followed by mixing uniformly and then tableting according to the conventional method, to obtain tablets.

The amount of Compound (1) to be contained as the active ingredient in a solid preparation varies according to the dosage form, method of administration, carrier, and age, body weight, etc. of the person who receives administration, and may be determined according to the individual conditions/circumstances and is not limited. The amount of Compound (1) is usually 0.1 to 20% (w/w), preferably 1 to 8% (w/w) of the total amount of the preparation. When the percentage is below 0.1% (w/w), pharmaceutical efficacy of the preparation cannot be expected, and when the percentage is above 20% (w/w), sufficient efficacy as solid dispersions cannot be exerted.

The amount of the water-soluble polymer to be combined in the solid preparation of the present invention is, for sufficient efficacy as solid dispersions, 3 to 100 weight parts, preferably 3 to 50 weight parts, more preferably 3 to 20 weight parts per unit weight of Compound (1) as the active ingredient; when polyvinylpyrrolidone is used, the amount is 3 to 20 weight parts, preferably 8 to 12 weight parts per unit weight of Compound (1); and when hydroxypropylcellulose is used, the amount is particularly desirably 6 to 15 weight parts per unit weight of Compound (1).

In order to obtain sufficient effect as the solid preparation of the present invention, the amount of the inorganic porous substance is desirably 4 to 14 weight parts, more desirably 6 to 13 weight parts, and further more desirably 7 to 11 weight parts per unit weight of Compound (1).

In order to obtain sufficient effect as the solid preparation of the present invention, the amount of the disintegrant is desirably 1 to 10 weight parts, particularly desirably 3 to 7 weight parts per unit weight of Compound (1) as the active ingredient. A desirable disintegrant is cross-linked polyvinylpyrrolidone (crospovidone).

The "solid preparation" prepared in this manner is "heavy" (high density), and can be used not only as tablets but also as an oral pharmaceutical preparation as it is. The solid preparation can also be made according to the conventional method into pharmaceutical preparations such as fine granules, microgranules, granules, capsules and liquid preparations.

When preparations such as powders, granules, and capsules are desired, such preparations are easily obtained by addition of a suitable excipient, disintegrant, lubricant, surfactant, etc. to the sized granules obtained with the above-mentioned method, followed by preparation according to the conventional method. These manufacture processes are well-known and common to those skilled in the art, and processes appropriate to the form of the preparation can be adopted.

When sugar-coated tablets or the like are desired, the surface of the tablet may be coated with an appropriate coating composition.

Because generally Compound (1) as a pharmaceutical active ingredient is slightly soluble or insoluble in water, the portion of the dose that is actually absorbed into blood is small so that the bioavailability is low, which is a drawback. The solid preparation of the present invention, however, is excellent in solubility, oral absorbability, and absorbability into blood, resulting in the remarkably improved bioavailability of Compound (1).

The solid preparation of the present invention not only has an improved solubility but also keeps an excellent stability.

The solid preparation of the present invention can be used concomitantly with another pharmaceutical preparation. For example, the solid preparation of the present invention containing Compound (1) as the active ingredient can be used concomitantly with another therapeutic agent for hyperlipidemia. In this case, the solid preparation of the present invention and the therapeutic agent for hyperlipidemia may be taken separately or used in the form of a combined drug containing both.

Preferable preparations of the agent for the prevention or treatment of the present invention include hard capsules and soft capsules prepared by filling the liquid, semi-solid or solid content obtained by dissolution of Compound (1) in a solvent followed by addition of an additive.

Hard capsules can be obtained, for example, by filling the powder containing Compound (1) or the above-mentioned granules in, for example, hard capsules (No. 00, No. 0, No. 1, No. 2, No. 3, No. 4, No. 5 capsules, etc.) molded with an edible film such as gelatin, pullulan and cellulose.

Soft capsules can be obtained, for example, by preparing a film of a certain thickness from the capsule base according to the conventional method, placing two sheets of the resultant film between symmetrical, rotating metal molds

to form an outer shell of soft capsules while dissolving Compound (1) in the solvent, filling the solution to which an additive is added as needed into the outer shell of the soft capsule, punching the outer shell of the soft capsules by, for example, rotation of the metal mold while sealing by welding, and drying.

The above-mentioned solvents include purified water, ethanol and vegetable oil, among which ethanol or a mixture of purified water and ethanol is desirable, and anhydrous ethanol is particularly desirable. The additives used usually for manufacture of capsules can be used without any restriction. These additives include propylene glycol esters of fatty acids; low molecular weight polyethylene glycol such as polyethylene glycol 200 to 600, glycerides thereof, and triglycerides of middle-chain fatty acids; alcohols/polyhydric alcohols and esters thereof such as stearyl alcohol, cetanol, and polyethylene glycol; fats and oils such as sesame oil, soybean oil, peanut oil, corn oil, hydrogenated oil, paraffin oil, and bleached beeswax; and fatty acids and derivatives thereof such as triethyl citrate, triacetin, stearic acid, palmitic acid, and myristic acid; which are suitable for preparation of liquid or semi-solid contents. For the capsules of the invention, propylene glycol esters of fatty acids are desirable as the additives. The propylene glycol esters of fatty acids are exemplified by propylene glycol monocaprylate (Capmul PG-8 (trade name), Sefol 218 (trade name), Capryol 90 (trade name)), propylene glycol monolaurate (Lauroglycol FCC (trade name)), propylene glycol monooleate (Myverol P-O6 (trade name)), propylene glycol myristate, propylene glycol monostearate, propylene glycol ricinoleate (Propymuls (trade name)), propylene glycol dicaprylate/dicaprate (Captex (registered trademark) 200 (trade name)), propylene glycol dilaurate, propylene glycol distearate and propylene glycol dioctanoate (Captex (registered trademark) 800 (trade name)). Substances used as the base of capsules of the invention are not limited but include natural polysaccharides such as agar, alginates, starch, xanthan and dextran; proteins such as gelatin and casein; sugar alcohols such as those derived from starch (for example corn starch); chemical treatment products such as hydroxyl starch, pullulan, hydroxypropylcellulose, hydroxypropylmethylcellulose, polyvinyl alcohol and the derivatives thereof, polyacrylic derivatives, polyvinylpyrrolidone and the derivatives thereof, and polyethylene glycol; and solvents such as purified water.

When the agent for the prevention or treatment of the present invention is a liquid preparation for oral administration, such as pharmaceutically acceptable emulsion, solution, suspension, syrup or elixir, examples of the diluents to be used include purified water, ethanol, vegetable oils and emulsifiers. In the liquid preparation, adjuvants such as infiltrating agents, suspensions, sweetening agents, aromatics, and antiseptics may be mixed in addition to the diluent.

When the agent for the prevention or treatment of the invention is a parenteral preparation such as injection, examples of the additives to be used include aseptic aqueous or nonaqueous solutions, solubilizers, suspensions and emulsifiers. Examples of the aqueous solutions, solubilizers and suspensions include distilled water for injection; isotonic sodium chloride solution, cyclodextrin and derivatives thereof; organic amines such as triethanolamine, diethanolamine, monoethanolamine and triethylamine, and inorganic alkali solutions. When an aqueous solution is used, propylene glycol, polyethylene glycol, a vegetable oil such as olive oil, or an alcohol such as ethanol may be added further. Examples of the solubilizers include surfactants such as polyoxyethylene hydrogenated castor oil, and sucrose fatty acid esters (mixed micelle formation), lecithine and hydrogenated lecithine (liposome formation). The parenteral preparation of the present invention can be made into an emulsion preparation according to the conventional method using a nonaqueous solvent such as a vegetable oil and an emulsifier such as lecithine, polyoxyethylene hydrogenated castor oil or poly (oxyethylene) poly(oxypropylene) glycol.

In this specification, diabetes means a disease with elevated blood sugar level, including type 1 diabetes and type 2 diabetes, and refers in particular to the type 2 diabetes. The type 2 diabetes is a disease in which entry of sugar into peripheral cells is decreased and thus blood sugar level is elevated. The causes therefor include decreased insulin secretion from pancreatic β cells and insulin resistance in peripheral cells (weakened insulin effect). In the type 2 diabetes accompanied by insulin resistance, strong insulin resistance will result in excessive insulin secretion, leading to a high blood insulin level. Compound (1), the active ingredient of the present invention, exerts a remarkable effect especially in type 2 diabetes accompanied with insulin resistance.

The surplus glucose in hyperglycemia accumulates in the form of lipid, and therefore type 2 diabetes can at the same time induce obesity (particularly visceral fat obesity). In addition, type 2 diabetes can also induce arteriosclerosis because the high blood sugar level increases the protein of vascular wall matrix and causes calcification. Compound (1), the active ingredient of the present invention, not only prevents or treats type 2 diabetes but also has a remarkable effect in prevention or treatment of obesity (particularly the visceral fat obesity) and arteriosclerosis which accompanies type 2 diabetes (especially type 2 diabetes accompanied by insulin resistance).

In the specification, obesity means obesity in which lipid accumulates in the viscera or subcutaneously. Compound (1), the active ingredient of the present invention, is remarkably effective in the visceral fat obesity in which lipid accumulates in the perivisceral region, for example around the greater omentum and mesenterium. When lipid is increased in viscera etc. , TNFα (tumor necrosis factor) or free fatty acids released from adipose cells weaken the effect of insulin in the peripheral cells, which causes insulin resistance, increase blood sugar level, and thus can induce type 2 diabetes. In visceral fat obesity, blood lipid level is also elevated, so that arteriosclerosis is a possible complication. Compound (1), the active ingredient of the present invention, not only prevents or treats obesity but also has a remarkable effect in prevention or treatment of type 2 diabetes (especially type 2 diabetes accompanied by insulin resistance) and

arteriosclerosis which accompany obesity (especially visceral fat obesity).

In the specification, arteriosclerosis means a disease in which the arterial wall is thickened with lipid accumulated on it and is hardened due to loss of elasticity. Compound (1), the active ingredient of the present invention, is remarkably effective for arteriosclerosis. Arteriosclerosis can be accompanied with type 2 diabetes (especially type 2 diabetes with insulin resistance) and obesity (especially visceral fat obesity). Compound (1), the active ingredient of the present invention, not only prevents or treats arteriosclerosis but also has a remarkable effect for the prevention or treatment of type 2 diabetes (especially type 2 diabetes accompanied by insulin resistance) and obesity (especially visceral fat obesity) accompanying arteriosclerosis.

The agent for the prevention or treatment of the present invention can be concomitantly used with another drug. Such drugs are exemplified by drugs for the prevention or treatment of hyperlipidemia, obesity or diabetes, which may be used separately or in combination of two or more drugs. Other drugs for treatment of hyperlipidemia include drugs of the statin series, in more concrete, lovastatin, simvastatin, pravastatin, fluvastatin, atorvastatin, and serivastatin. Other drugs for the treatment of obesity include mazindol and orlistat. Other drugs for the treatment of diabetes include insulin preparations, sulfonylurea agents, insulin secretion-promoting agents, sulfonamide agents, biguanide agents, α-glucosidase inhibitors and insulin resistance-improving agents, in more concrete, insulin, glibenclamide, tolbutamide, glyclopyramide, acetohexamide, glimepiride, tolazamide, gliclazide, nateglinide, glybuzole, metformin hydrochloride, buformin hydrochloride, voglibose, acarbose and pioglitazone hydrochloride.

Timing of administration of the agent for the prevention or treatment of the present invention and the concomitant drug are not limited, and they may be administered to the person who receives administration concomitantly or with a time lag. The dose of the concomitant drug is in accordance with the clinical dose, and can be selected appropriately according to the person who receives administration, age and body weight of the person, symptoms, time of administration, dosage form, method of administration, combination, etc. The dosage form of the concomitant drug is not limited as far as the agent for the prevention or treatment of the invention and the concomitant drug are administered in combination.

The invention is explained in detail in the following examples and tests, but is not limited thereto.

Example 1

1. Granulation;

(1) Dissolution of Compound (1) as active ingredient; 30.0 g of Compound (1) was added to an acetone-anhydrous ethanol mixture obtained by mixing with stirring of 418.5 g of acetone and 46.5 g of anhydrous ethanol (weight % ratio: 9 to 1) in a propeller mixer, and then the mixture was thoroughly dissolved.

(2) Dissolution of polymer; 300.0 g of polyvinylpyrrolidone (Povidone K30) as a water-soluble polymer was gradually added into the above-mentioned solvent where Compound (1) was dissolved, and the mixture was dissolved by further mixing with stirring.

(3) Preparation of excipient;

Separately, 239.1 g of light anhydrous silicic acid as an excipient and 150.0 g of calcium carboxymethylcellulose as a disintegrant were uniformly mixed by agitation in a high-shear mixer/granulator. (4) Mixing of excipient with polymer solution;

An organic mixed solvent in which Compound (1) prepared in the above step (2) and polyvinylpyrrolidone had been dissolved was gradually added to the mixture of light anhydrous silicic acid prepared in the above step (3), and the mixture was mixed by stirring until it became homogenous. (5) Crude granulation;

The mixture obtained in the above step (4) was granulated according to the conventional method and then dried in a vacuum drier. (6) Granular classification;

In order to make a uniform granule size, granular classification was performed according to the conventional method using a sieve of 600μm in aperture.

2. Tableting;

The above-mentioned granulation process was repeated twice, and to 1438.2 g of the resultant granules were added 150.0 g of cross-linked polyvinylpyrrolidone (crospovidone) as a disintegrant and 7.8 g of magnesium stearate as a lubricant. The mixture was uniformly blended using a double cone blender, followed by tableting using a rotary tableting machine, to produce about 1,200 tablets weighing each about 133 mg (containing about 5 mg/tablet of Compound (1) as an active ingredient).

Example 2

A film of a certain thickness was prepared from a film composition (1a) according to the conventional method. Two sheets of the resultant film were placed between symmetrical, rotating metal molds to form an outer shell of soft capsules while a content solution (1b) was filled into the outer shell of the soft capsules, and capsule preparations were prepared by punching, while sealing by welding, the outer shell of the soft capsules through rotation of the metal mold. These capsules were dried in a rotary drier and further dried by allowing to stand for 4 days, thereby to give soft capsules (major axis of about 19.5 mm and minor axis of about 7 mm).

(Table 1)

| | |
|---|---|
| (1a) Film composition | |
| Gelatin | 100 mass parts |
| Corn starch-derived sugar alcohol solution | 30 mass parts |
| Purified water | 100 mass parts |
| (1b) Content liquid (per capsule) | |
| Compound (1) | 5 mg |
| Propyleneglycol fatty acid ester | 277 mg |
| Ethanol | 148 mg |

Example 3

Soft capsules (major axis of about 19.5 mm and minor axis of about 7 mm) were obtained similarly according to Example 1 except that the film composition (1a) and the content liquid (1b) were replaced with the following film composition (2a) and the following content liquid (2b), respectively.

(Table 2)

| | |
|---|---|
| (2a) Film composition | |
| Gelatin | 202.2 mg |
| Corn starch-derived sugar alcohol solution | 60.6 mg |
| Purified water | sufficient quantity |
| (2b) Content liquid (per capsule) | |
| Compound (1) | 5 mg |
| Propyleneglycol fatty acid ester | 280 mg |
| Anhydrous ethanol | 120 mg at the most |

Example 4

Compound (1), an excipient and a binder were mixed to prepare a granulated powder according to the conventional method. The resultant granulated powder was mixed with a disintegrant and a lubricant to prepare granules for tableting according to the conventional method. The resultant granules for tableting was tableted according to the conventional method to give tablets. Specific formulation examples were shown below.

Example 4-1

(Table 3)

| | |
|---|---|
| Compound (1) | 5 mg |
| Lactose | 133.06 mg |
| Crystalline cellulose | 18 mg |
| Hydroxypropyl methylcellulose 2910 | 5.4 mg |
| Crospovidone | 18 mg |
| Magnesium stearate | 0.54 mg |

Example 4-2

(Table 4)

| | |
|---|---|
| Compound (1) | 5 mg |
| Lactose | 92.44 mg |
| Corn starch | 15 mg |
| Hydroxypropyl methylcellulose 2910 | 3.6 mg |
| Carboxymethyl starch | 3.6 mg |
| Magnesium stearate | 0.36 mg |

Example 4-3

(Table 5)

| | |
|---|---|
| Compound (1) | 5 mg |
| D-Mannitol | 158.4 mg |
| Hydroxypropyl methylcellulose 2910 | 6 mg |
| Calcium silicate | 20 mg |
| Crospovidone | 10 mg |
| Magnesium stearate | 0.6 mg |

Test Example 1

Antidiabetic effect

Male KKAy mice (Clea Japan, Inc.) aged 7 weeks which are genetically diabetic (insulin-resistant) were used in the test. Blood was previously collected from the orbital venous plexus of animals acclimated to feeding with normal powdered diet (CRF-1, Oriental Yeast, Co., Ltd.), and then the animals were grouped into 5 groups containing 6 animals each so that there might be no intergroup difference with respect to plasma parameters (neutral fat value, free fatty acid value, insulin value, blood sugar level). Compound (1) was added to the powdered diet so that the concentration in the diet might be 1, 3, 10, and 30 mg/kg/day. The animals were allowed to take the diet ad libitum for 14 days, and blood was collected again from the orbital venous plexus in the morning on day 15 for the measurement of the above-mentioned plasma parameters. The results are shown in Table 6 below.

(Table 6)

| Dose (mg/kg/day) | Neutral fat (mg/dl) | Free fatty acid (mEq/l) | Insulin (ng/ml) | Blood sugar (mg/dl) |
|---|---|---|---|---|
| Control | 699±26.7 | 368±11.6 | 144±13.2 | 545±16.3 |
| 1 | 676±57.2 | 353±15.9 | 117±14.0 | 413±34.9 |
| 3 | 716±79.9 | 293±11.7 | 103±10.5 | 516±14.9 |
| 10 | 545±53.1 | 235±24.9 | 102±11.3 | 405±36.8 |
| 30 | 298±21.0 | 171±17.4 | 67±10.4 | 331±39.6 |

The results shown above indicate clearly that Compound (1) is excellent in reduction of neutral fats, which also leads to reduction of free fatty acids that induce insulin resistance responsible for diabetes. Namely, Compound (1) improves insulin sensitivity to make insulin effective, so that the necessary amount of insulin is decreased and the insulin value is also decreased. In conclusion, reduced neutral fat makes it possible to decrease blood sugar level, and thus Compound (1) is useful as an agent for the prevention or treatment of diabetes.

Test Example 2

Anti-obesity effect

Japanese white rabbits (Kitayama Labes., Co Ltd.) aged 9 weeks were used in the test. Blood was collected from the auricular artery of animals acclimated to feeding with high fat diet (RC-4 containing 0.3% cholesterol and 3% peanut oil, supplied by Oriental Yeast, Co., Ltd.), and then the animals were grouped into 4 groups containing 12 animals each so that there might be no intergroup difference with respect to body weight and plasma parameters (neutral fat value, total cholesterol value, HDL cholesterol value). Compound (1) was added to the high fat diet so that the intake of Compound (1) might be 3, 10, and 30 mg/kg/100 g diet/day. The animals were given the diet restricted to 100 g diet/day for 12 weeks, and body weight and its gain were determined after the 12 weeks. The results are shown in Table 7 below.

(Table 7)

| Dose (mg/kg/day) | Body weight (g) | Body weight gain (g) |
|---|---|---|
| Control | 3143.7 | 1196.3 |
| 3 | 3122.1 | 1176.0 |
| 10 | 2997.1 | 1065.9 |
| 30 | 2969.0 | 1025.4 |

The results demonstrate that Compound (1) is excellent in suppressing effect on body weight gain even under such condition that there is no difference in intake of diet. Thus Compound (1) is useful for the prevention or treatment of obesity.

Test Example 3

Visceral fat-decreasing effect

SD rats (Charles River Japan, Inc.) aged 8 weeks were used in the test. Animals acclimated to feeding with 35% (w/w) high fat diet for 1 week were grouped into 2 groups, i.e. a control group and a group treated with Compound (1), for the test. Animals in the control group received the 35% (w/w) high fat diet, and those in the Compound (1) group received the diet prepared by adding Compound (1) to 0.029% (w/w) of the 35% (w/w) high fat diet, for 3 months. Tap water was available ad libitum to the animals in the test.

Fat weight was determined 3 months later with an X ray CT scanner for laboratory animals (Aloka). The results are shown in Table 8 below.

(Table 8)

| | Visceral fat weight (g) | Subcutaneous fat weight (g) | Muscle weight (g) |
|---|---|---|---|
| Control group | 71.0 | 34.2 | 198.9 |
| Compound (1) group | 28.8 | 14.9 | 204.4 |

In contrast to the control group, the Compound (1) group, showed decrease of visceral fat and subcutaneous fat without decreasing the muscle weight. Fat weight decrease was especially remarkable in visceral fat.

The compound related to the present invention, as compared with the control, showed fat weight-decreasing effect without decreasing muscle weight. Therefore the results indicate that Compound (1) is useful as an agent for the prevention or treatment of obesity.

Test Example 4

Anti-arteriosclerosis effect

Japanese white rabbits (Kitayama Labes., Co Ltd.) aged 9 weeks were used in the test. Blood was collected from the auricular artery of animals acclimated to feeding with high fat diet (RC-4 containing 0.3% cholesterol and 3% peanut oil, Oriental Yeast, Co., Ltd.), and then the animals were grouped into 4 groups containing 12 animals each so

that there might be no intergroup difference with respect to body weight and parameters in plasma (neutral fat value, total cholesterol value, HDL cholesterol value). Compound (1) was added to the high fat diet so that the intake of Compound (1) might be 3, 10, and 30 mg/kg/100 g diet/day. The animals were allowed to take the diet restricted to 100 g diet/day for 12 weeks, lipid of the aorta was stained, and the stained region was defined as the arteriosclerotic region. The percentage of area of the arteriosclerotic region was calculated according to the equation below. The results are shown in Table 9 below.

Percentage of area of arteriosclerotic region (%) =

$$\frac{\text{(area of arteriosclerotic region)}}{\text{(total area of the region assessed)}} \times 100$$

(Table 9)

| Dose (mg/kg/day) | Percentage of area of arteriosclerotic region (%) |
|---|---|
| Control | 46.2 |
| 3 | 28.4 |
| 10 | 7.7 |
| 30 | 0.15 |

The above-mentioned test results demonstrate that Compound (1), even under the condition that there is no difference in cholesterol intake, suppresses remarkably the deposit of lipid in the aorta. Namely Compound (1) suppresses formation of arteriosclerotic region in the aorta to decrease cardiovascular events. Therefore Compound (1) is useful for the prevention or treatment of arteriosclerosis.

Industrial Applicability

The agent for the prevention or treatment of this invention is useful for the treatment or prevention of diabetes, obesity, or arteriosclerosis.

## Claims

1. An agent for the prevention or treatment of diabetes, comprising a compound represented by the formula (1):

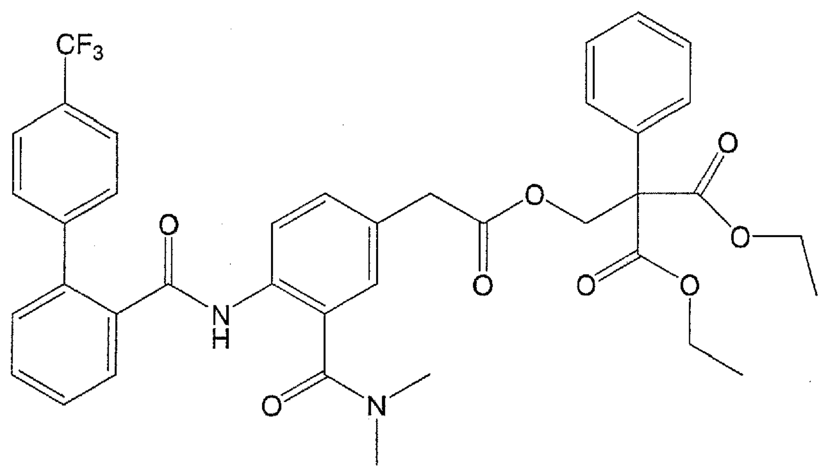

(1)

or its pharmaceutically acceptable salt as an active ingredient.

2. The agent for the prevention or treatment according to claim 1, wherein the diabetes is non-insulin dependent

diabetes mellitus.

3. The agent for the prevention or treatment according to claim 2, wherein the non-insulin dependent diabetes mellitus is accompanied by obesity or/and arteriosclerosis.

4. The agent for the prevention or treatment according to claim 3, which suppresses obesity or/and arteriosclerosis.

5. The agent for the prevention or treatment according to claim 3 or 4, wherein the obesity is visceral fat obesity.

6. The agent for the prevention or treatment according to any one of claims 1 to 5, which causes no induction of liver dysfunction.

7. The agent for the prevention or treatment according to any one of claims 1 to 6, wherein the unit dose of the compound represented by the formula (1) or its pharmaceuticallyacceptable salt is 0.5 to 30 mg and the daily dose is 0.5 to 30 mg.

8. An agent for the prevention or treatment of obesity, comprising a compound represented by the formula (1):

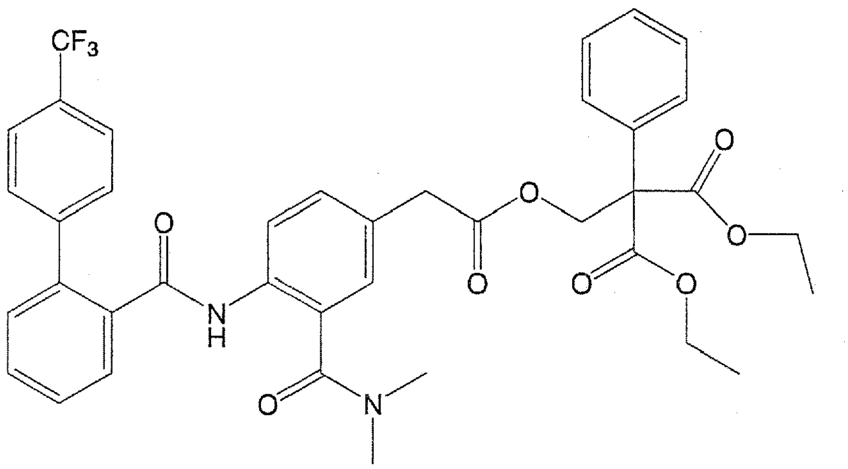

(1)

or its pharmaceutically acceptable salt as an active ingredient.

9. The agent for the prevention or treatment according to claim 8, wherein the obesity is visceral fat obesity.

10. The agent for the prevention or treatment according to claim 9, wherein the visceral fat obesity is accompanied by non-insulin dependent diabetes mellitus or/and arteriosclerosis.

11. The agent for the prevention or treatment according to claim 10, which suppresses non-insulin dependent diabetes mellitus or/and arteriosclerosis.

12. The agent for the prevention or treatment according to any one of claims 8 to 11, which causes no induction of liver dysfunction.

13. The agent for the prevention or treatment according to any one of claims 8 to 12, wherein the unit dose of the compound represented by the formula (1) or its pharmaceutically acceptable salt is 0.5 to 30 mg and the daily dose is 0.5 to 30 mg.

14. An agent for the prevention or treatment of arteriosclerosis, comprising a compound represented by the formula (1):

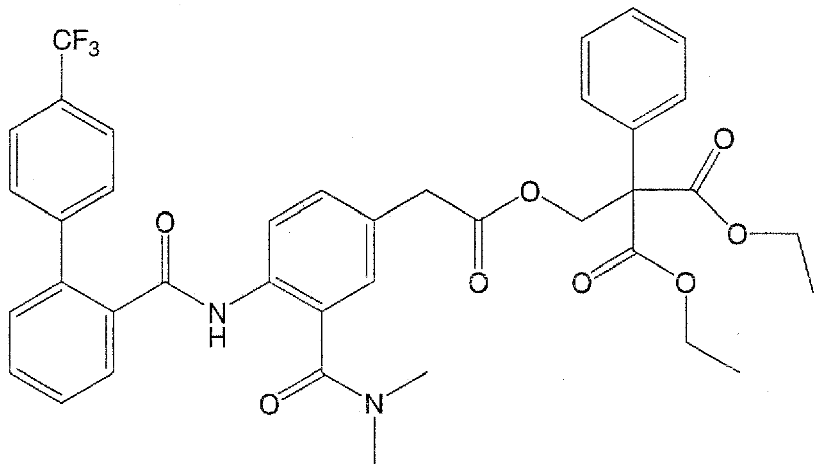

(1)

or its pharmaceutically acceptable salt as an active ingredient.

**15.** The agent for the prevention or treatment according to claim 14, wherein the arteriosclerosis is accompanied by non-insulin dependent diabetes mellitus or/and visceral fat obesity.

**16.** The agent for the prevention or treatment according to claim 15, which suppresses non-insulin dependent diabetes mellitus or/and visceral fat obesity.

**17.** The agent for the prevention or treatment according to any one of claims 14 to 16, which causes no induction of liver dysfunction.

**18.** The agent for the prevention or treatment according to any one of claims 14 to 17, wherein the unit dose of the compound represented by the formula (1) or its pharmaceutically acceptable salt is 0.5 to 30 mg and the daily dose is 0.5 to 30 mg.

**19.** Use of a compound represented by the formula (1):

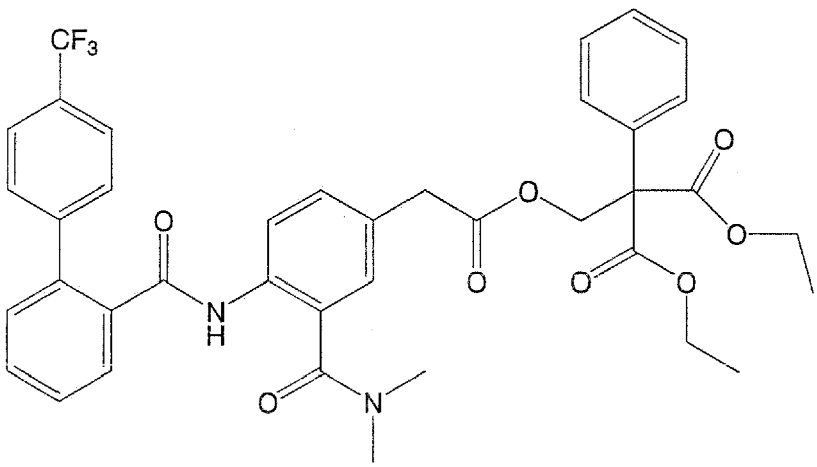

(1)

or its pharmaceutically acceptable salt for preparing a medicine for the prevention or treatment of diabetes.

**20.** The use according to claim 19, wherein the diabetes is non-insulin dependent diabetes mellitus.

**21.** The use according to claim 20, wherein the non-insulin dependent diabetes mellitus is accompanied by obesity or/and arteriosclerosis.

**22.** The use according to claim 21, which suppresses obesity or/and arteriosclerosis.

**23.** The use according to claim 21 or 22, wherein the obesity is visceral fat obesity.

**24.** The use according to any one of claims 19 to 23, which causes no induction of liver dysfunction.

**25.** The use according to any one of claims 19 to 24, wherein the unit dose of the compound represented by the formula (1) or its pharmaceutically acceptable salt is 0.5 to 30 mg and the daily dose is 0.5 to 30 mg.

**26.** Use of a compound represented by the formula (1):

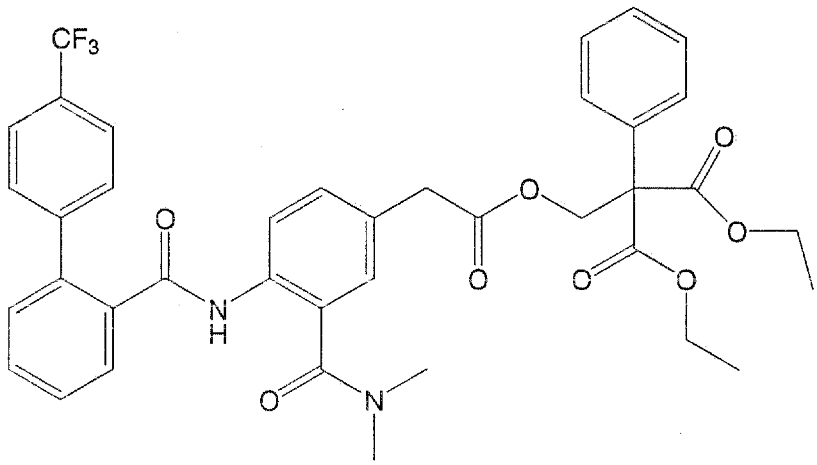

(1)

or its pharmaceutically acceptable salt for preparing a medicine for the prevention or treatment of obesity.

**27.** The use according to claim 26, wherein the obesity is visceral fat obesity.

**28.** The use according to claim 27, wherein the visceral fat obesity is accompanied by non-insulin dependent diabetes mellitus or/and arteriosclerosis.

**29.** The use according to claim 28, which suppresses non-insulin dependent diabetes mellitus or/and arteriosclerosis.

**30.** The use according to any one of claims 26 to 29, which causes no induction of liver dysfunction.

**31.** The use according to any one of claims 26 to 30, wherein the unit dose of the compound represented by the formula (1) or its pharmaceutically acceptable salt is 0.5 to 30 mg and the daily dose is 0.5 to 30 mg.

**32.** Use of a compound represented by the formula (1):

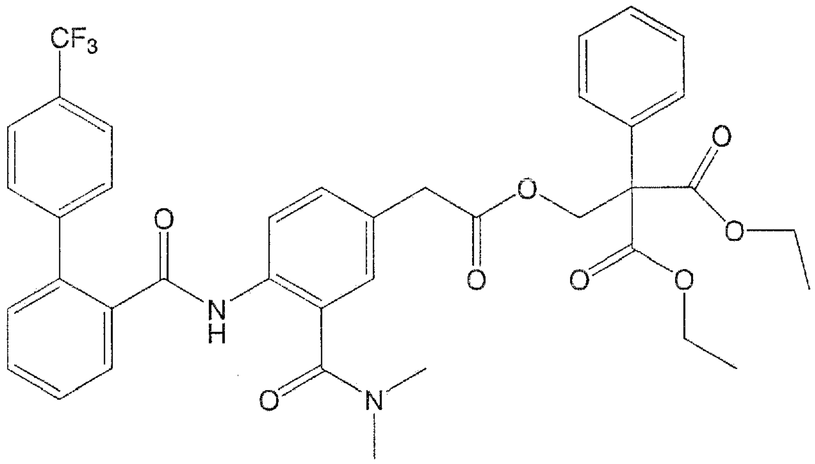

(1)

or its pharmaceutically acceptable salt for preparing a medicine for the prevention or treatment of arteriosclerosis.

**33.** The use according to claim 32, wherein the arteriosclerosis is accompanied by non-insulin dependent diabetes mellitus or/and visceral fat obesity.

**34.** The use according to claim 33, which suppresses non-insulin dependent diabetes mellitus or/and arteriosclerosis.

**35.** The use according to any one of claims 32 to 34, which causes no induction of liver dysfunction.

**36.** The use according to any one of claims 32 to 35, wherein the unit dose of the compound represented by the formula (1) or its pharmaceutically acceptable salt is 0.5 to 30 mg and the daily dose is 0.5 to 30 mg.

Europäisches Patentamt
European Patent Office
Office européen des brevets

# EUROPEAN SEARCH REPORT

Application Number
EP 11 18 0387

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | EP 1 479 666 A (JAPAN TOBACCO INC [JP]) 24 November 2004 (2004-11-24)<br>* page 54, line 5 - line 37 *<br>* page 126; examples 2-5; table 18 *<br>* pages 208-209, paragraph 547 *<br>----- | 1-36 | INV.<br>A61K31/216<br>A61P3/04<br>A61P3/10<br>A61P9/10 |
| X | JP 2003 321424 A (JAPAN TOBACCO INC) 11 November 2003 (2003-11-11)<br>* paragraph [0001] *<br>* paragraph [0268] *<br>* paragraph [0318] *<br>* paragraph [0323] *<br>* examples 2-5; table 18 *<br>----- | 1-36 | |

TECHNICAL FIELDS SEARCHED (IPC)

A61K

The present search report has been drawn up for all claims

1

| Place of search | Date of completion of the search | Examiner |
|---|---|---|
| Munich | 20 October 2011 | Terenzi, Carla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 18 0387

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2011

| Patent document cited in search report | Publication date | Patent family member(s) | Publication date |
|---|---|---|---|
| EP 1479666 A | 24-11-2004 | AU 2003211617 A1 | 09-09-2003 |
| | | BR 0306292 A | 24-08-2004 |
| | | CA 2460682 A1 | 04-09-2003 |
| | | CN 1630629 A | 22-06-2005 |
| | | CN 1943786 A | 11-04-2007 |
| | | CN 101838218 A | 22-09-2010 |
| | | CO 5570695 A2 | 31-10-2005 |
| | | WO 03072532 A1 | 04-09-2003 |
| | | JP 2010043098 A | 25-02-2010 |
| | | KR 20060053023 A | 19-05-2006 |
| | | MX PA04002602 A | 11-08-2004 |
| | | NO 20041872 A | 06-05-2004 |
| | | NZ 531890 A | 24-02-2006 |
| | | RU 2293721 C2 | 20-02-2007 |
| | | SG 165154 A1 | 28-10-2010 |
| | | US 2005075367 A1 | 07-04-2005 |
| | | US 2010158996 A1 | 24-06-2010 |
| | | ZA 200402275 A | 23-04-2005 |
| | | ZA 200502495 A | 20-09-2005 |
| | | ZA 200502496 A | 12-10-2005 |
| JP 2003321424 A | 11-11-2003 | JP 3662566 B2 | 22-06-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 20030725322 A